# NEUE EUROPÄISCHE PATENTSCHRIFT

(11) **EP 1 856 024 B2**
(45) Veröffentlichungstag und Bekanntmachung des Hinweises auf die Entscheidung über den Einspruch: **17.08.2011**
(45) Hinweis auf die Patenterteilung: 06.08.2008
(21) Anmeldenummer: 06708604.1
(22) Anmeldetag: 02.03.2006
(51) Int. Cl.: C07C 201/12, C07C 205/12

(54) **VERFAHREN ZUR HERSTELLUNG SUBSTITUIERTER BIPHENYLE**
METHOD FOR PRODUCING SUBSTITUTED BIPHENYLS
PROCEDE DE FABRICATION DE BIPHENYLS SUBSTITUES

(30) Priorität: 02.03.2005 DE 102005010107
(43) Veröffentlichungstag der Anmeldung: 21.11.2007
(73) Patentinhaber: BASF SE, 67056 Ludwigshafen (DE)
(72) Erfinder: ENGEL, Stefan, 55268 Nieder-Olm (DE); OBERDING, Tanja, 12620-000 Piquete-Sp-Brasil (BR)
(86) Internationale Anmeldenummer: PCT/EP2006/060400
(87) Internationale Veröffentlichungsnummer: WO 2006/092429

(56) Entgegenhaltungen:
- EP-A1- 1 285 924
- WO-A-97/33846
- WO-A1-97/33846
- JP-A- 2001 055 360
- JP-A- 2003 119 175

## Beschreibung

Die vorliegende Erfindung betrifft ein Verfahren zur Herstellung substituierter Biphenyle der Formel I 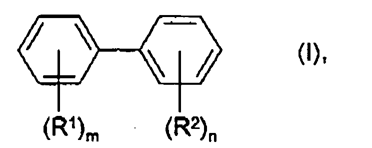 in der die Substituenten folgende Bedeutungen haben:
- R¹: Nitro, Amino oder NHR³;
- R²: Halogen;
- R³: C₁-C₄-Alkyl, C₂-C₄-Alkenyl oder C₂-C₄-Alkinyl;
- m: 1 oder 2, wobei im Falle von m = 2 die beiden Reste R¹ verschiedene Bedeutun- gen haben können;
- n: 1, 2 oder 3, wobei im Falle von n = 2 oder 3 die Reste R² verschiedene Bedeu- tungen haben können;
dadurch gekennzeichnet, dass man eine Verbindung der Formel II 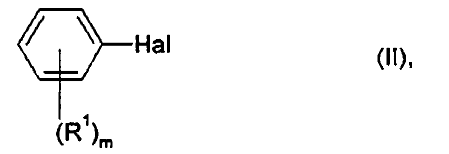 worin Hal für Halogen steht und R¹ und m die oben angegebenen Bedeutungen haben, in Gegenwart einer Base und eines Palladium-Katalysators ausgewählt aus der Gruppe: a) Palladium-Triaryl- oder Trialkylphosphinkomplex mit Palladium in der Oxidationsstufe Null, b) Salz des Palladiums in Gegenwart von Triaryl- oder Trialkylphosphin als Komplexligand oder c) gegebenenfalls auf Träger aufgezogenes metallisches Palladium,
in Gegenwart von Triaryl- oder Trialkylphosphin in einem Lösungsmittel mit einer Diphenylborinsäure (III) 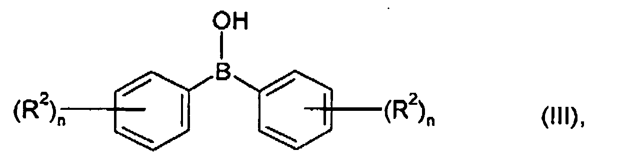 worin R² und n die oben angegebenen Bedeutungen haben, umsetzt,
wobei die verwendeten Triaryl- oder Trialkylphosphine substituiert sein können.

Tetrahedron Lett. 32, Seite 2277 (1991) ist zu entnehmen, dass die Kupplungsreaktion zwischen Phenylboronsäure und Chlorbenzol bei Verwendung des Katalysators [1,4-bis-(Diphenylphosphan)butan]palladium(II)dichlorid mit einer Ausbeute von lediglich 28 % verläuft

Aus der EP-A 0 888 261 ist ein Verfahren zur Herstellung von Nitrobiphenylen durch Umsetzung von Chlornitrobenzolen mit einer Phenylboronsäure in Gegenwart eines Palladium-Katalysators und einer Base bekannt. Bei diesem Verfahren ist eine sehr hohe Katalysatorkonzentration nötig.

Der vorliegenden Erfindung lag daher die Aufgabe zugrunde, ein wirtschaftliches und im industriellen Maßstab technisch umsetzbares Verfahren zur regioselektiven Herstellung von substituierten Biphenylen bereitzustellen, welches mit einer verringerten Palladium-Katalysatorkonzentration arbeitet.

Demgemäss wurde das eingangs definierte Verfahren gefunden.

Die Diphenylborinsäure (III) wird durch Reaktion von gegebenenfalls substituiertem Phenylmagnesiumchlorid V mit Trialkylborat, bevorzugt Trimethylborat, in Tetrahydrofuran als Lösungsmittel gemäß folgendem Schema 1 erhalten.

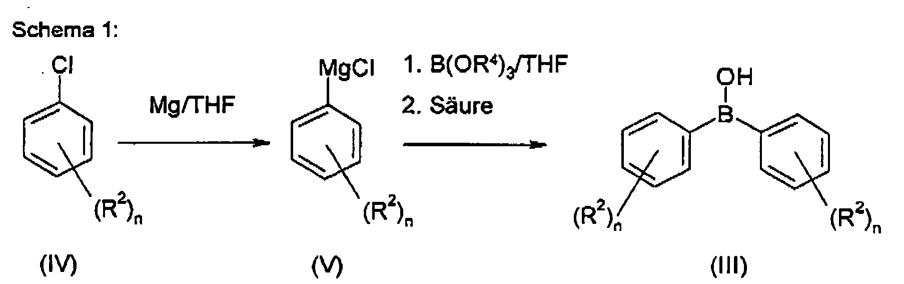

R⁴ steht für C₁-C₄-Alkyl, bevorzugt Methyl.

Wesentlich für eine hohe Ausbeute von Diphenylborinsäure (III) ist der Einsatz von nur 0.7 eq. Trialkylborat, bezogen auf das eingesetzte Chlorbenzol (IV). Bei einer Einsatzzahl von etwa 1.1 eq Trialkylborat entsteht Phenylboronsäure, wie in der EP-A 0 888 261 beschrieben.

Diese Reduktion der Trialkylborat-Einsatzzahl hat mehrere überraschende Vorteile in Bezug auf die Herstellung von Nitrobiphenylen (I). Die Raum/Zeit-Ausbeute wird erhöht. Die Einsatzstoffkosten durch Reduzierung des teuren Trimethylborates werden erniedrigt. Die Diphenylborinsäuren (III) sind in Tetrahydrofuran löslich im Gegensatz zu den in der EP-A 0 888 261 verwendeten Phenylboronsäuren, was zu einer Verbesserung der Wärmeabfuhr während der Reaktion führt, der mit einem geringeren Verbrauch der Kühlkapazität einhergeht Dies führt wiederum zu einer höheren Verfahrenssicherheit.

Die Reaktionstemperatur bei dieser Verfahrensstufe liegt bei 10 bis 30°C, bevorzugt bei 15 bis 25°C.

Die nach dem vorliegenden Verfahren hergestellten substituierten Biphenyle haben folgende bevorzugte Substituenten:
- R¹: Nitro, Amino, Methylamino, Propylamino, Butylamino, Allylamino oder Propargylamino, besonders bevorzugt Nitro, Amino oder Methylamino, ganz besonders bevorzugt Nitro oder Amino;
- R²: Fluor, Chlor oder Brom, ganz besonders bevorzugt Fluor oder Chlor;
- R³: Methyl, Ethyl, Propyl, Butyl, Allyl oder Propargyl, besonders bevorzugt Methyl, Ethyl oder Allyl, ganz besonders bevorzugt Methyl;
- m: 1;
- n: 1 oder 2. bevorzugt 1.

Die Durchführung der sich anschließenden homogen katalysierten Suzuki Biaryl-Kreuzkupplung erfolgt nach Schema 2.

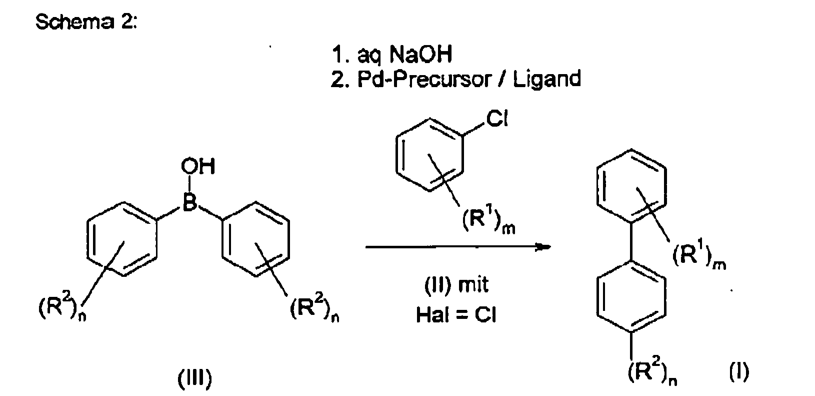

Dabei geht man bevorzugt von Diphenylborinsäuren der Formel (III) aus, in denen R² und n die oben angegebenen Bedeutungen haben.

Darüber hinaus sind Diphenylborinsäuren (III) als Ausgangsmaterial bevorzugt, in denen n insbesondere für 1 steht.

Ganz besonders bevorzugt sind Di-(4-fluorphenyl)-borinsäure und vor allem Di-(4-chlorphenyl)borinsäure als Ausgangsverbindung (III).

Vorzugsweise geht man von Verbindungen (II) aus, welche eine einzige Nitro- oder Aminogruppe tragen (m = 1), insbesondere 4-Nitrochlorbenzol oder 4-Aminochlorbenzol und vor allem 2-Nitrochlorbenzol oder 2-Aminochlorbenzol.

Die Verbindung (II) wird, bezogen auf die Diphenylborinsäuren (III) (Diphenylborinsäure-Äquivalente), normalerweise äquimolar, bevorzugt mit bis zu 20-prozentigem, vorzugsweise mit bis zu 50-prozentigem, Überschuss eingesetzt.

Als Base können organische Basen, z. B. tertiäre Amine, eingesetzt werden. Bevorzugt verwendet man z. B. Triethylamin oder Dimethylcyclohexylamin.

Als Base verwendet man vorzugsweise Alkalimetallhydroxide, Erdalkatimetallhydroxide, Alkalimetallcarbonate, Erdalkalimetallcarbonate, Alkalimetallhydrogencarbonate, Alkalimetallacetate, Erdalkalimetallacetate, Alkalimetallalkoholate und Erdalkalimetallalkoholate, im Gemisch und insbesondere einzeln.

Als Base besonders bevorzugt sind Alkalimetallhydroxide, Erdalkalimetallhydroxide, Alkalimetallcarbonate, Erdalkalimetallcarbonate und Alkalimetallhydrogencarbonate.

Als Base insbesondere bevorzugt sind Alkalimetallhydroxide, z. B. Natriumhydroxid und Kaliumhydroxid, sowie Alkalimetallcarbonate und Alkolimetallhydrogencarbonate, z. B. Lithiumcarbonat, Natriumcarbonat und Kaliumcarbonat.

Die Base wird im erfindungsgemäßen Verfahren vorzugsweise mit einem Anteil von 100 bis 500 Mol-%, besonders bevorzugt 150 bis 400 Mol-%, bezogen auf die Diphenylborinsäuren (III), eingesetzt.

Geeignete Palladium-Katalysatoren sind Palladiurn-Ligandenkomplexe mit Palladium in der Oxidationsstufe Null, Salze des Palladiums in Gegenwart von Komplexliganden oder gegebenenfalls auf Träger aufgezogenes metallisches Palladium, vorzugsweise in Gegenwart von Komplexliganden.

Geeignete Komplexliganden sind neutrale Liganden wie Triaryl- und Trialkylphosphine, welche in den Arylringen gegebenenfalls substituiert sein können, wie Triphenylphosphin (TPP), Di-1-adamantyl-n-butylphosphin, Tri-ferf.-butylphosphin (TtBP) oder 2-(Dicyclohexylphosphino)-biphenyl.

Darüber hinaus sind in der Literatur auch weitere besonders reaktive Komplexliganden aus anderen Strukturklassen beschrieben worden, wie unter anderem 1,3-Bis-(2,6-diisopropylphenyl)-4,5-H2-imidazoliumchlorid (vgl. z. B. G. A. Grasa et al., Organometallics 2002, 21, 2866) und Tris-(2,4-di-*tert.*-butylphenyl)-phosphit (vgl. A. Zapf et al., Chem. Eur. J. 2000, 6, 1830).

Die Reaktivität der Komplexliganden kann durch Zusatz eines quartären Ammoniumsalzes wie Tetra-n-butylammoniumbromid (TBAB) gesteigert werden (vgl. z. B. D. Zim et al., Tetrahedron Lett. 2000, 41, 8199).

Bei Bedarf kann die Wasserlöslichkeit der Palladium-Komplexe durch verschiedene Substituenten verbessert werden, wie Sulfonsäure- oder Sulfonsäuresalzgruppen, Carbonsäure- oder Carbonsäuresalzgruppen, Phosphonsäure-, Phosphonium- oder Phosphonsäuresatzgruppen, Peralkylammonium-, Hydroxy- und Polyethergruppen.

Aus den Palladium-Ligandenkomplexen mit Palladium in der Oxidationsstufe 0 verwendet man vorzugsweise Tetrakis(triphenylphosphin)palladium und daneben Tetrakis[tri(o-tolyl)phosphin]palladium.

In den Salzen des Palladiums, welche in Gegenwart von Komplexliganden verwendet werden, liegt das Palladium normalerweise in der zweifach positiven Oxidationsstufe vor. Bevorzugt verwendet man Palladiumchlorid, Palladiumacetat oder Bisacetonitrilpalladiumchlorid, Besonders bevorzugt wird Palladiumchlorid verwendet.

In der Regel werden 6 bis 60, bevorzugt 15 bis 25, Äquivalente der vorstehend genannten Komplexliganden, insbesondere Triphenylphosphin und Tri-*tert.*-butyl-phosphin, mit einem Äquivalent des Palladiumsalzes kombiniert.

In der EP-A 0 888 261 wird die Verwendung von 2 bis 6 Äquivalenten Triphenylphosphin pro Äquivalent des Palladium-Katalysators beschrieben. Die Verwendung hoher Ligandenüberschüsse wird in der Literatur allgemein als unvorteilhaft betrachtet, da dadurch eine Inaktivierung des katalytisch aktiven Komplexes erwartet wird (vgl. z. B. J. Hassan et al., Chem. Rev. 2002, 102, 1359).

Somit war es überraschend, dass der hohe Überschuss an Komplexliganden in Kombination mit dem geringen Katalysatoreinsatz zu einer Erhöhung der Gesamtausbeute des Verfahrens der vorliegenden Erfindung und damit verbunden zu einer Verbesserung der Wirtschaftlichkeit führte.

Metallisches Palladium verwendet man vorzugsweise in pulverisierter Form oder auf einem Trägermaterial, z. B. als Palladium auf Aktivkohle, Palladium auf Aluminiumoxid, Palladium auf Bariumcarbonat, Palladium auf Bariumsulfat, Palladium auf Calciumcarbonat, Palladium auf Aluminiumsilikaten wie Montmorillonit, Palladium auf SiO₂ und Palladium auf Calciumcarbonat, jeweils mit einem Palladiumgehalt von 0,5 bis 12 Gew.%. Diese Katalysatoren können neben Palladium und dem Trägermaterial weitere Dotierstoffe, z. B. Blei, enthalten.

Besonders bevorzugt ist bei der Verwendung von gegebenenfalls auf Träger aufgezogenem, metallischem Palladium die Mitverwendung der vorstehend genannten Komplexliganden, insbesondere die Verwendung von Palladium auf Aktivkohle in Gegenwart von Triphenylphosphin als Komplexligand, wobei die Phenylgruppen im Triphenylphosphin vorzugsweise mit insgesamt ein bis drei Sulfonatgruppen substituiert sind.

Der Palladiumkatalysator wird im erfindungsgemäßen Verfahren mit einem niedrigen Anteil von 0,001 bis 1,0 Mol-%, vorzugsweise von 0,005 bis 0,5 Mol% oder von 0,01 bis 0,5 Mol% und insbesondere von 0,005 bis 0,05 Mol%, bezogen auf die Verbindung (II) eingesetzt.

Der geringe Einsatz eines Palladiumsalzes in Kombination mit einer hohen Einsatzzahl an Komplexliganden stellen einen wesentlichen Kostenvorteil dieses Verfahrens gegenüber den Verfahren des Stands der Technik dar.

Das erfindungsgemäße Verfahren kann in einem Zweiphasensystem aus wässriger Phase und fester Phase, d. h. dem Katalysator, durchgeführt werden. Die wässrige Phase kann dabei neben Wasser auch ein wasserlösliches organisches Lösungsmittel enthalten.

Für das erfindungsgemäße Verfahren geeignete organische Lösungsmittel sind Ether wie Dimethoxyethan, Diethylenglykoldimethylether, Tetrahydrofuran, Dioxan und *tert.-*Butylmethylether, Kohlenwasserstoffe wie n-Hexan, n-Heptan, Cyclohexan, Benzol, Toluol und Xylol, Alkohole wie Methanol, Ethanol, 1-Propanol, 2-Propanol, Ethylenglykol, 1-Butanol, 2-Butanol und *tert.*-Butanol, Ketone wie Aceton, Ethylmethylketon und iso-Butylmethylketon, Amide wie Dimethylformamid, Dimethylacetamid und N-Methylpyrrolidon, jeweils einzeln oder in Mischung.

Bevorzugte Lösungsmittel sind Ether wie Dimethoxyethan, Tetrahydrofuran und Dioxan, Kohlenwasserstoffe wie Cyclohexan, Toluol und Xylol, Alkohole wie Ethanol, 1-Propanol, 2-Propanol, 1-Butanol und *tert.*-Butanol, jeweils einzeln oder in Mischung.

In einer besonders bevorzugten Variante werden im erfindungsgemäßen Verfahren Wasser, ein oder mehrere in Wasser unlösliche und ein oder mehrere in Wasser lösliche Lösungsmittel eingesetzt, beispielsweise Mischungen aus Wasser und Dioxan oder Wasser und Tetrahydrofuran oder Wasser, Dioxan und Ethanol oder Wasser, Tetrahydrofuran und Methanol oder Wasser, Toluol und Tetrahydrofuran, vorzugsweise Wasser und Tetrahydrofuran oder Wasser, Tetrahydrofuran und Methanol.

Die Gesamtmenge an Lösungsmittel liegt normalerweise bei 3000 bis 500 und vorzugsweise bei 2000 bis 700 g pro Mol der Verbindung (II).

Zweckmäßigerweise werden zur Durchführung des Verfahrens die Verbindung (II), die Diphenylborinsäuren (III), die Base sowie die katalytische Menge des Palladium-Katalysators in eine Mischung aus Wasser und einem oder mehreren inerten organischen Lösungsmitteln gegeben und bei einer Temperatur von 50°C bis 120°C, vorzugsweise 70°C bis 110°C, besonders bevorzugt 90°C bis 100°C, für einen Zeitraum von 1 bis 50, vorzugsweise 2 bis 24 Stunden gerührt.

Je nach verwendetem Lösungsmittel und Temperatur stellt sich ein Druck von 1 bar bis 6 bar ein, bevorzugt 1 bar bis 4 bar.

Bevorzugt wird die Reaktion in Wasser und Tetrahydrofuran durchgeführt.

Die Durchführung kann in üblichen für derartige Verfahren geeigneten Apparaturen erfolgen.

Nach beendeter Umsetzung wird als Feststoff anfallender Palladium-Katalysator beispielsweise durch Filtration abgetrennt und das Rohprodukt vom Lösungsmittel bzw. den Lösungsmitten befreit.

Bei nicht völlig wasserlöslichen Produkten werden wasserlösliche Palladium-Katalysatoren oder Komplexliganden bei der Trennung der Wasserphase vom Rohprodukt vollstandig abgetrennt.

Anschließend kann nach dem Fachmann bekannten und dem jeweiligen Produkt angemessenen Methoden, z. B. durch Umkristallisation, Destillation, Sublimation, Zonenschmelzen, Schmelzkristallisation oder Chromatographie weiter aufgereinigt werden.

Mit dem erfindungsgemäßen Verfahren lassen sich beispielsweise herstellen:
4'-Chlor-2-nitrobiphenyl,
4'-Chlor-2-aminobiphenyl,
4'-Fluor-2-nitrobiphenyl,
4'-Fluor-2-aminobiphenyl,
4'-Brom-2-nitrobiphenyl,
4'-Brom-2-aminobiphenyl,
3'-Fluor-2-nitrobiphenyl,
3'-Fluor-2-aminobiphenyl,
3'-Chlor-2-nitrobiphenyl,
3'-Chlor-2-aminobiphenyl,
3'-Brom-2-nitrobiphenyl,
3'-Brom-2-aminobiphenyl,
4'-Fluor-nitrobiphenyl,
4'-Fluor-4-aminobiphenyl,
4'-Chlor-4-nitrobiphenyl,
4'-Chlor-4-aminobiphenyl,
4'-Brom-4-nitrobiphenyl,
4'-Brom-4-aminobiphenyl.

Das erfindungsgemäße Verfahren liefert die Verbindungen I in sehr hohen bis zu quantitativen Ausbeuten bei sehr guter Reinheit.

Die mit dem erfindungsgemäßen Verfahren erhältlichen Biphenyle eignen sich als Vorprodukte für substituierte Biphenylamine, welche ihrerseits Zwischenprodukte für fungizide Pflanzenschutz-Wirkstoffe (vgl. z. B. EP-A 545 099) sind.

### Synthese von 4'-Chlor-2-nitrobiphenyl

### Beispiel 1: Di-(4-Chlorphenyl)-borinsäure

Eine Lösung aus 120 g Trimethylborat und 590 g Tetrahydrofuran wurde auf 11 °C gekühlt. Dazu wurden 1000 g einer 20 Gew.-%igen Lösung aus 4-Chlorphenylmagnesiumchlorid in Tetrahydrofuran in 2 Stunden dosiert. Dabei stellte sich eine Temperatur von 20 - 21 °C ein. Nach vollständiger Zugabe wurde die Reaktionslösung noch 1 Stunde bei 20°C gerührt.

Die Reaktionsmischung wurde anschließend mit 621 g 10%iger, wässriger Salzsäure behandelt und 30 Minuten bei 40°C gerührt Nach Phasentrennung erhielt man 1500 g einer Lösung von Di-(4-chlorphenyl)-borinsäure in Tetrahydrofuran (Umsatz 87 %). Die organische Phase kann als Rohprodukt weiter verarbeitet oder Di-(4-chlorphenyl)-borinsäure kann durch Säulenchromatographie an Kieselgel mit Mischungen aus Essigester und Cyclohexan isoliert werden.

### Beispiel 2: Reaktion von Di-(4-chlorphenyl)-borinsäure und 1-Chlor-2-nitrobenzol

In einem Autoklaven wurden 240 g einer 20 Gew.-%igen, wässrigen Natronlauge bei 15 - 20°C vorgelegt. Dazu wurden 539 g einer 9 - 10 Gew.-%igen Lösung von Di-(4-chlorphenyl)-borinsäure in Dioxan bei 18 - 22°C in 26 Minuten dosiert. Nach vollständiger Zugabe wurde die Reaktionslösung 40 Minuten bei 18 - 22°C gerührt. Zu der Reaktionslösung wurden 2,4 g einer 50 Gew.-%igen Lösung von Triphenylphosphin in Dioxan gegeben. Nach vollständiger Zugabe wurde die Reaktionslösung 30 Minuten bei 18 - 22°C gerührt. Schließlich wurden 117 mg (Bisacetonitril)-palladium(II)chlorid und 84 g 1-Chlor-2-nitrobenzol zu der Reaktionslösung gegeben. Die Reaktionslösung wurde für 11,5 Stunden auf 100°C aufgeheizt. Dabei stellte sich im Druckbehälter ein Überdruck von 3,7 bar ein.

Nach vollständiger Umsetzung der Di-(4-chlorphenyl)-borinsäure wurde die Reaktionslösung auf 40 - 45°C abgefühlt und der Druckbehälter auf Normaldruck entspannt. Die Reaktionslösung wurde mit 250 g 10 Gew.-%iger, wässriger Salzsäure extrahiert. Nach Phasentrennung erhielt man eine Lösung von 4-Chlor-2'-nitrobiphenyl in Dioxan (Umsatz 99 %). Dioxan wurde durch Destillation im Vakuum entfernt und 4-Chlor-2'-nitrobiphenyl konnte durch Schmelzkristallisation isoliert werden.

### Beispiel 3: Reaktion von Di-(4-chlorphenyl)-borinsäure und 1-Chlor-2-nitrobenzol

in einem Autoklaven wurden 495 g Gew.-20 %ige, wässrige Natronlauge bei 15 - 20°C vorgelegt. Dazu wurden 1000 g einer 11 Gew.%igen Lösung von Di-(4-chlorphenyl)-borinsäure in Tetrahydrofuran bei 18 - 22°C in 30 Minuten dosiert. Nach vollständiger Zugabe wurde die Reaktionslösung 30 Minuten bei 18 - 22°C gerührt. Zu der Reaktionslösung wurden 3,5 g einer 50 Gew.%igen Lösung von Triphenylphosphin in Tetrahydrofuran gegeben. Nach vollständiger Zugabe wurde die Reaktionslösung 30 Minuten bei 20-21°C gerührt. Schließlich wurden 0,9 g Palladium(II)chlorid in 227 g geschmolzenem 1-Chlor-2-nitrobenzol zu der Reaktionslösung gegeben. Die Reaktionslösung wurde für 6 - 8 Stunden auf 100°C aufgeheizt. Dabei stellte sich im Autoklaven ein Überdruck von 3,0 bar ein.

Nach vollständiger Umsetzung der Di-(4-chlorphenyl)-borinsäure wurde der Autoklav auf Normaldruck entspannt und die Reaktionslösung auf 40 - 50°C abgekühlt Die Reaktionslösung wurde mit 450 g 10 Gew.-%iger, wässriger Salzsäure extrahiert. Nach Phasentrennung erhielt man eine Lösung von 4-Chlor-2'-nitrobiphenyl in Tetrahydrofuran (Umsatz 99 %).

### Beispiel 4: Reaktion von Di-(4-chlorphenyl)-borinsäure und 1-Chlor-2-nitrobenzol

In einem 4 1-Vierhalskolben wurden 770 g 22 Gew.-%ige, wässrige Natronlauge bei 20°C vorgelegt. Dazu wurden 2045 g einer 13 Gew.-%igen Lösung von Di-(4-chlorphenyl)-borinsäure in Tetrahydrofuran bei 20°C in 30 Minuten dosiert. Nach vollständiger Zugabe wurde die Reaktionslösung 30 Minuten bei 20°C gerührt. Zu der Reaktionslösung wurden 9.8 g Triphenylphosphin, 1,7 g Palladium(II)chlorid und 273 g geschmolzenes 1-Chlor-2-nitrobenzol gegeben. Die Reaktionslösung wurde für 20 Stunden auf Rückflusstemperatur aufgeheizt.

Nach vollständiger Umsetzung der 4-Chlorphenylboronsäure wurde die Reaktionslösung auf 40°C abgekühlt und anschließend mit 255 g 35 Gew.%iger, wässriger Salzsäure extrahiert. Nach Phasentrennung erhielt man eine Lösung von 4-Chlor-2'-nitrabiphenyl in Tetrahydrofuran (Umsatz 99 %).

### Beispiel 5: Reaktion von 4-Chlorphenylboronsäure und 1-Chlor-2-nitrobenzol

In einem 4 m³-Reaktor wurden 1773 kg einer 13 Gew.%igen Lösung von 4-Chlorphenylboronsäure in Tetrahydrofuran bei 18 - 22°C vorgelegt. In 20 Minuten wurden 538 kg 25 Gew.-%ige, wässrige Natronlauge und 140 kg Wasser unter Rühren bei 22 - 30°C dosiert. Nach vollständiger Zugabe wurde die Reaktionslösung 30 Minuten bei 22 - 25°C gerührt. Zu der Reaktionslösung wurden 2.28 kg Triphenylphosphin, 372 g Palladium(II)chlorid und 252 kg geschmolzenes 1-Chlor-2-nitrobenzol gegeben. Die Reaktionslösung wurde für 18 Std. auf 66°C aufgeheizt. Nach vollständiger Umsetzung der 4-Chlorphenylboronsäure wurde die Reaktionslösung auf 45°C abgekühlt und mit 794 kg 10 Gew.%iger, wässriger Salzsäure extrahiert. Nach Phasentrennung erhielt man eine Lösung von 4-Chlor-2'-nitrobiphenyl in Tetrahydrofuran (Umsatz 99 %).

### Beispiel 6: Reaktion von Di-(4-chlorphenyl)-borinsäure und 1-Chlor-2-nitrobenzol

In einem Autoklaven wurden 177 g einer 20 Gew.%igen, wässrigen Natronlauge bei 15°C vorgelegt. Dazu wurden 415 g einer 9-10 Gew.-%igen Lösung von Di-(4-chlorphenyl)-borinsäure in Tetrahydrofuran bei 18-20°C in 30 Minuten dosiert. Nach vollständiger Zugabe wurde die Reaktionslösung 30 Minuten bei 18 - 20°C gerührt. Zu der Reaktionslösung wurden 0,24 g einer 50 Gew.-%igen Lösung von Tri-*tert.*-butylphosphin in Tetrahydrofuran gegeben. Nach vollständiger Zugabe wurde die Reaktionslösung 30 Minuten bei 18 - 20°C gerührt. Schließlich wurden 104 mg einer 10 Gew.-%igen Lösung von Palladium(II)chlorid in 10 Gew.-%iger, wässriger Salzsäure und 91 g einer 85 Gew.-%igen Lösung von 1-Chlor-2-nitrobenzol in Tetrahydrofuran zu der Reaktionslösung gegeben. Die Reaktionslösung wurde für 12 Stunden auf 100°C aufgeheizt. Dabei stellte sich ein Überdruck von 3,5 bar ein.

Nach vollständiger Umsetzung der Di-(4-chlorphenyl)-borinsäure wurde die Reaktionslösung auf 40 - 50°C abgefühlt und der Druckbehälter auf Normaldruck entspannt. Die Reaktionslösung wurde mit 125 g 10 Gew.-%iger, wässriger Salzsäure extrahiert. Nach Phasentrennung erhielt man eine Lösung von 4-Chlor-2'-nitrobiphenyl in Tetrahydrofuran (Umsatz 85 %).

### Beispiel 7: Reaktion von Di-(4-Norphenyl)-borinsäure und 1-Brom-2-anilin

In einem Autoklaven wurden 240 g einer 20 Gew.%igen, wässrigen Natronlauge bei 20°C vorgelegt Dazu wurden 539 g einer 9-10 Gew.-%igen Lösung von Di-(4-chlorphenyl)-borinsäure in Tetrahydrofuran bei 20°C in 30 Minuten dosiert. Nach vollständiger Zugabe wurde die Reaktionslösung 30 Minuten bei 20°C gerührt. Zu der Reaktionslösung wurden 1,3 g einer 50 Gew.-%igen Lösung von Triphenylphosphin in Tetrahydrofuran gegeben. Nach vollständiger Zugabe wurde die Reaktionslösung 30 Minuten bei 20°C gerührt. Schließlich wurden 320 mg einer 10 Gew.-%igen Lösung von Palladium(II)chlorid in 10 Gew.-%iger Salzsäure und 108 g einer 85 Gew.-%igen Lösung von 1-Brom-2-anilin In Tetrahydrofuran zu der Reaktionslösung gegeben. Die Reaktionslösung wurde für 12 Stunden auf 100°C aufgeheizt. Dabei stellte sich im Druckbehälter ein Überdruck von 3,5 bar ein.

Nach vollständiger Umsetzung der Di-(4-chlorphenyl)-borinsäure wurde die Reaktionslösung auf 40 - 50°C abgekühlt und der Druckbehälter auf Normaldruck entspannt Nach Phasentrennung wurde die organische Phase mit 100 g 20 Gew.-%iger, wässriger Natronlauge extrahiert. Man erhielt eine Lösung von 4-Chlor-2'-aminobiphenyl in Tetrahydrofuran (Umsatz 85 %). Tetrahydrofuran wurde durch Destillation im Vakuum entfernt und 4-Chlor-2'-aminobiphenyl konnte durch Kristallisation isoliert werden.

## Patentansprüche

1. Verfahren zur Herstellung substituierter Biphenyle der Formel **I** 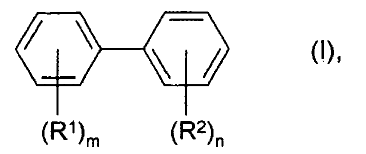 in der die Substituenten folgende Bedeutungen haben:
R¹ Nitro, Amino oder NHR³;
R² Halogen;
R³ C₁-C₄-Alkyl, C₂-C₄-Alkenyl oder C₂-C₄-Alkinyl;
m 1 oder 2, wobei im Falle von m = 2 die beiden Reste R¹ verschiedene Be- deutungen haben können;
n 1, 2 oder 3, wobei im Falle von n = 2 oder 3 die Reste R² verschiedene Be- deutungen haben können;
**dadurch gekennzeichnet, dass** man eine Verbindung der Formel II 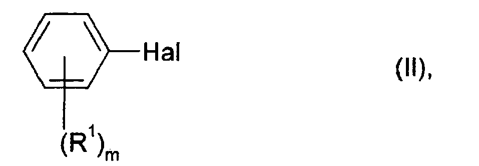 worin Hal für Halogen steht und R¹ und m die oben angegebenen Bedeutungen haben, in Gegenwart einer Base und eines Palladium-Katalysators ausgewählt aus der Gruppe: a) Palladium-Triaryl- oder Trialkylphosphinkomplex mit Palladium in der Oxidationsstufe Null, b) Salz des Palladiums in Gegenwart von Triaryl- oder Trialkylphosphin als Komplexligand oder c) gegebenenfalls auf Träger aufgezogenes metallisches Palladium,
in Gegenwart von Triaryl- oder Trialkylphosphin in einem Lösungsmittel mit einer Diphenylborinsäure (III) 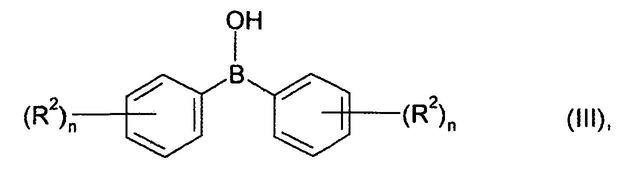 worin R² und n die oben angegebenen Bedeutungen haben, umsetzt,
wobei die verwendeten Triaryl- oder Trialkylphosphine substituiert sein können.

2. Verfahren nach Anspruch 1, **dadurch gekennzeichnet, dass** man als Verbindung (II) 2-Nitrochlorbenzol einsetzt.

3. Verfahren nach Anspruch 1 oder 2, **dadurch gekennzeichnet, dass** man von einer Diphenylborinsäure, welche nur in der 4-Position substituiert ist, als Verbindung (III) ausgeht.

4. Verfahren nach den Ansprüchen 1 oder 2, **dadurch gekennzeichnet, dass** man eine Diphenylborinsäure (III) einsetzt, welche als einzigen Substituenten in der 4-Position Fluor oder Chlor trägt.

5. Verfahren nach den Ansprüchen 1 oder 2, **dadurch gekennzeichnet, dass** man von Di-(4-chlorphenyl)-borinsäure als Verbindung (III) ausgeht.

6. Verfahren nach den Ansprüchen 1 bis 5, **dadurch gekennzeichnet, dass** man als Palladium-Katalysator a) gemäß Anspruch 1 Tetrakis(triphenylphosphin)palladium oder Tetrakis(tri-tert.butylphosphin)palladium verwendet.

7. Verfahren nach den Ansprüchen 1 bis 5, **dadurch gekennzeichnet, dass** man einen Palladium-Katalysator b) gemäß Anspruch 1 verwendet.

8. Verfahren nach den Ansprüchen 1 bis 5, **dadurch gekennzeichnet, dass** man als Palladium-Katalysator c) gemäß Anspruch 1 metallisches Palladium auf Aktivkohle in Gegenwart von Triphenylphosphin verwendet, dessen Phenylgruppen mit insgesamt 1 bis 3 Sulfonatgruppen substituiert sind.

9. Verfahren nach Anspruch 7, **dadurch gekennzeichnet, dass** man als Salz des Palladium-Katalysators b), Palladiumchlorid, Palladiumacetat oder Bisacetonitrilpalladiumchlorid verwendet.

10. Verfahren nach Anspruch 7, **dadurch gekennzeichnet, dass** man einen Palladium-Katalysator b) verwendet, bei dem pro Äquivalent des Palladiumsalzes 6 bis 60 Äquivalente Triphenylphosphin verwendet werden.

11. Verfahren nach Anspruch 1, **dadurch gekennzeichnet, dass** man 0,001 bis 1,0 mol-% des Palladium-Katalysators, bezogen auf die Verbindung (11), verwendet.

12. Verfahren nach Anspruch 1, **dadurch gekennzeichnet, dass** man die Umsetzung bei einer Temperatur von 50 bis 120°C durchführt.

13. Verfahren nach Anspruch 1, **dadurch gekennzeichnet, dass** man die Umsetzung in einem Gemisch aus Wasser und einem organischen Lösungsmittel durchführt.

14. Verfahren nach Anspruch 13, **dadurch gekennzeichnet, dass** man einen Ether als organisches Lösungsmittel verwendet.

15. Verfahren nach Anspruch 1, **dadurch gekennzeichnet, dass** man die Umsetzungen bei einem Druck von 1 bis 6 bar durchführt.

## Claims

1. A process for preparing substituted biphenyls of the formula I 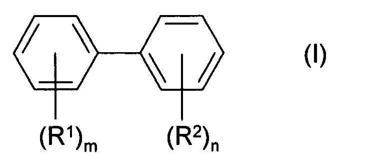 in which the substituents are defined as follows:
R¹ is nitro, amino or NHR₃,
R² is halogen,
R³ is C₁-C₄-alkyl, C₂-C₄-alkenyl or C₂-C₄-alkynyl,
m is 1 or 2, where, in the case that m = 2, the two R¹ radicals may have different definitions,
n is 1, 2 or 3, where, in the case that n = 2 or 3, the two R² radicals may have different definitions,
which comprises reacting a compound of the formula II 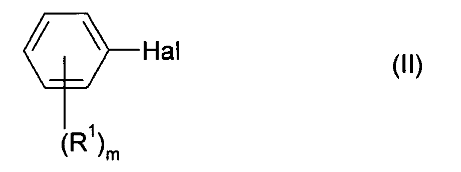 in which Hal is halogen and R¹ and m are each as defined above, in the presence of a base and of a palladium catalyst selected from the group of: a) palladium-triarylphosphine or -trialkylphosphine complex with palladium in the zero oxidation state, b) salt of palladium in the presence of triarylphospine or trialkylphosphine as a complex ligand or c) metallic palladium applied to support if appropriate,
in the presence of triarylphosphine or trialkylphosphine, in a solvent, with a diphenylborinic acid (III) 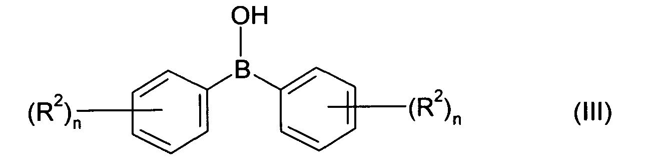 in which R² and n are each as defined above, where the triarylphosphines or trialkylphosphines used may be substituted.

2. The process according to claim 1, wherein the compound (II) used is 2-nitrochlorobenzene.

3. The process according to claim 1 or 2, wherein the starting compound (III) is a diphenylborinic acid which is substituted only in the 4-position.

4. The process according to claim 1 or 2, wherein a diphenylborinic acid (III) is used which bears, as the sole substituent in the 4-position, fluorine or chlorine.

5. The process according to claim 1 or 2, wherein the starting compound (III) is di(4-chlorophenyl)borinic acid.

6. The process according to claims 1 to 5, wherein the palladium catalyst a) according to claim 1 used is tetrakis(triphenylphosphine)palladium or tetrakis(tri-tert-butylphosphine)palladium.

7. The process according to claims 1 to 5, wherein a palladium catalyst b) according to claim 1 is used.

8. The process according to claims 1 to 5, wherein the palladium catalyst c) according to claim 1 used is metallic palladium on activated carbon in the presence of triphenylphosphine whose phenyl groups are substituted by a total of from 1 to 3 sulfonate groups.

9. The process according to claim 7, wherein the salt of the palladium catalyst b) used is palladium chloride, palladium acetate or bisacetonitrilepalladium chloride.

10. The process according to claim 7, wherein a palladium catalyst b) is used for which from 6 to 60 equivalents of triphenylphosphine are used per equivalent of the palladium salt.

11. The process according to claim 1, wherein from 0.001 to 1.0 mol% of the palladium catalyst is used, based on the compound (II).

12. The process according to claim 1, wherein the reaction is carried out at a temperature of from 50 to 120°C.

13. The process according to claim 1, wherein the reaction is carried out in a mixture of water and an organic solvent.

14. The process according to claim 3, wherein the organic solvent used is an ether.

15. The process according to claim 1, wherein the reactions are carried out at a pressure of from 1 to 6 bar.

## Revendications

1. Procédé de fabrication de biphényles substitués de formule I : 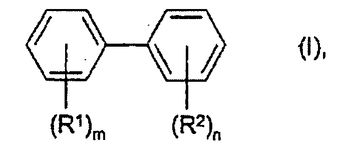 dans laquelle les substituants ont les significations suivantes :
R¹ représente un groupement nitro, amino ou NHR³ ;
R² représente un halogène;
R³ représente un groupement alkyle en C₁-C₄, alcényle en C₂-C₄ ou alcynyle en C₂-C₄ ;
m est égal à 1 ou 2 et, dans le cas où m = 2, les deux radicaux R¹ peuvent avoir des significations différentes ;
n est égal à 1, 2 ou 3 et, dans le cas où n = 2 ou 3, les radicaux R² peuvent avoir des significations différenties ;
**caractérisé en ce que** l'on fait réagir un composé de formule II : 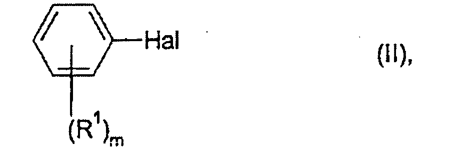 dans laquelle Hal représente un halogène et R¹ et m ont les significations indiquées ci-dessus, en présence d'une base et d'un catalyseur de palladium, choisi dans le groupe constitué : a) d'un complexe de palladium-triarylphosphine ou de palladium-trialkylphosphine avec du palladium au degré d'oxydation zéro, b) d'un sel du palladium en présence de triarylphosphine ou de trialkylphosphine sous forme de ligand complexé ou c) éventuellement, de palladium sous forme de métal déposé sur un support,
en présence de triarylphosphine ou de trialkylphosphine dans un solvant avec un acide diphénylborique (III) : 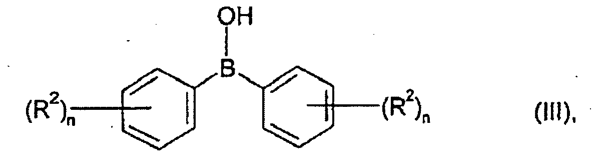 dans lequel R² et n ont les significations indiquées ci-dessus,
les triarylphosphines ou les trialkylphosphines utilisées pouvant être substituées.

2. Procédé selon la revendication 1, **caractérisé en ce que** l'on utilise du 2-nitrochlorobenzène comme composé (II).

3. Procédé selon la revendication 1 ou 2, **caractérisé en ce que** l'on part, comme composé (III), d'un acide diphénylborique, qui est substitué seulement en position 4.

4. Procédé selon les revendications 1 ou 2, **caractérisé en ce que** l'on utilise un acide diphénylborique (III) qui porte, comme seuls substituants en position 4, un fluor ou un chlore.

5. Procédé selon les revendications 1 ou 2, **caractérisé en ce que** l'on part de l'acide di-(4-chlorophényl)-borïque comme composé (III).

6. Procédé selon les revendications 1 à 5, **caractérisé en ce que** l'on utilise, comme catalyseur de palladium a) selon la revendication 1, du tétrakis(triphénylphosphine)-palladium ou du tétrakis(tri-tert-butylphosphine)-palladium.

7. Procédé selon les revendications 1 à 5, **caractérisé en ce que** l'on utilise un catalyseur de palladium b) selon la revendication 1.

8. Procédé selon les revendications 1 à 5, **caractérisé en ce que** l'on utilise comme catalyseur de palladium c) selon la revendication 1, du palladium sous forme de métal déposé sur du charbon actif, en présence de triphénylphosphine, dont les groupements phényle sont substitués par, au total, 1 à 3 groupements sulfonate.

9. Procédé selon la revendication 7, **caractérisé en ce que** l'on utilise, comme sel du catalyseur de palladium b), du chlorure de palladium, de l'acétate de palladium ou du chlorure de bis-acétonitrile-palladium.

10. Procédé selon la revendication 7, **caractérisé en ce que** l'on emploie un catalyseur de palladium b), dans lequel on utilise 6 à 60 équivalents de
triphénylphosphine par équivalent du sel de palladium.

11. Procédé selon la revendication 1, **caractérisé en ce que** l'on utilise 0,001 à 1,0 % en mole du catalyseur de palladium par rapport au composé (II).

12. Procédé selon la revendication 1, **caractérisé en ce que** l'on réalise la réaction à une température de 50 à 120 °C.

13. Procédé selon la revendication 1, **caractérisé en ce que** l'on effectue la réaction dans un mélange constitué d'eau et d'un solvant organique.

14. Procédé selon la revendication 13, **caractérisé en ce que** l'on utilise un éther comme solvant organique.

15. Procédé selon la revendication 1, **caractérisé en ce que** l'on effectue les réactions sous une pression de 1 à 6 bars.
